# EUROPEAN PATENT SPECIFICATION

(11) **EP 4 231 972 B1**
(45) Date of publication and mention of the grant of the patent: **04.03.2026**
(21) Application number: 21802814.0
(22) Date of filing: 19.10.2021
(51) Int. Cl.: A61F 2/64, A61F 2/70, F15B 9/04

(54) **PROSTHESIS FOR LIMBS OF THE HUMAN BODY AND ELECTROHYDRAULIC ACTUATOR FOR SUCH PROSTHESIS**
PROTHESE FÜR GLIEDMASSEN DES MENSCHLICHEN KÖRPERS UND ELEKTROHYDRAULISCHER AKTUATOR FÜR EINE SOLCHE PROTHESE
PROTHÈSE POUR MEMBRES DU CORPS HUMAIN ET ACTIONNEUR ÉLECTROHYDRAULIQUE POUR LADITE PROTHÈSE

(30) Priority: 22.10.2020 IT 202000025039
(43) Date of publication of application: 30.08.2023
(73) Proprietor: Fondazione Istituto Italiano di Tecnologia, 16163 Genova (GE) (IT); I.N.A.I.L. ISTITUTO NAZIONALE PER L'ASSICURAZIONE CONTRO GLI INFORTUNI SUL LAVORO, 00144 Roma (IT); Politecnico Di Torino, 10129 Torino (TO) (IT)
(72) Inventor: TESSARI, Federico, 00062 Bracciano (RM) (IT); LAFFRANCHI, Matteo, 16163 Genova (GE) (IT); DE MICHIELI, Lorenzo, 16163 Genova (GE) (IT); GALLUZZI, Renato, 10129 Torino (TO) (IT); AMATI, Nicola, 10129 Torino (TO) (IT); TONOLI, Andrea, 10129 Torino (TO) (IT)
(74) Representative: Metroconsult Srl
(86) International application number: PCT/IB2021/059611
(87) International publication number: WO 2022/084847

(56) References cited:
- WO-A2-2014/016583
- CN-A- 110 202 608
- CN-B- 110 043 519
- US-A- 3 521 729
- US-A- 5 888 212
- US-A1- 2010 023 133
- US-B2- 10 087 962

## Description

The present invention generally relates to prostheses for limbs of people who have suffered amputations or disabilities.

As is known, in recent years the field of physical rehabilitation, and more generally the field of devices aiding the human body, has seen very important developments due to the application of modern technologies and know-how to various equipment and materials employed for making prostheses, which integrate or replace parts of the human body that have been maimed or amputated during traumatic events.

This field is, therefore, very important for public healthcare or accident prevention services, such as those provided by hospitals or accident prevention and insurance organizations, and especially for all those people who need to regain body functionality by means of artificial prosthetic devices.

In particular, the invention concerns limb prostheses, particularly prostheses for the lower limbs, but also for the upper limbs, involving articular movement.

The development of such prostheses is currently tending towards increased customization, i.e. they are made to size for the individual patient who will have to wear the prosthetic device.

This applies to the dimensional characteristics of the prosthesis, because it clearly has to anatomically adapt itself to the body of the person using it, and also to its functional characteristics, i.e. those aspects which relate to the behaviour of the artificial prosthetic device, which must reproduce the behaviour of the missing natural limb.

In this context, many aspects have to be taken into account, such as, for example, the shape of the various parts of the prosthesis (which must adapt itself to the body part with which it is engaged), the materials it is made of, and, as far as the limbs discussed herein are concerned, the active and resistant force it exerts following the movements imparted by the user to the artificial limb.

For a better understanding of this last point, it is necessary to consider the recent technological advances and trends of the industry, which are increasingly moving towards the making of prostheses that are electromechanical devices co-operating with the user in order to facilitate his/her movements and increase his/her performance by ensuring improved capabilities.

For example, in the case of lower-limb prostheses involving also the knee joint, the prosthetic device includes two distinct and mutually articulated portions: the first one is applied to the end of a thigh amputated above the knee, while the second reproduces the tibial tract of the leg, with the ankle and the foot.

The prosthetic device thus constructed must support the steps of the user during the various phases of the gait cycle, which may differ according to the circumstances: for example, one may walk slower or faster, or take shorter or longer strides, e.g. when walking uphill or on level ground.

For this reason, the most advanced prosthetic devices have a behaviour that may be either active or passive depending on the operating phase being executed, during which they exert, respectively, either a motive action or a resistant action during the knee extension and flexion phases.

To this end, the prosthetic devices known in the art typically comprise one or more actuators, springs and other electro-mechanical components (pumps, valves, batteries, gears, etc.), which can operate in an actuated or semi-actuated manner in order to effect the prosthesis' movements.

One example of this state of the art is described in American patent application published under reference number US 2010/0023133 (by Fairbanks et al.), which relates to a semi-actuated transfemoral prosthetic knee.

The device described in this document is essentially a lower-limb prosthesis that comprises a hydraulic actuator which is active between the upper first portion applied to the thigh and the lower second portion of the tibial tract including the foot, which are mutually connected to reproduce the knee joint.

The actuator supplies a predefined force during a part of the flexion and/or extension of the prosthetic knee joint, which is necessary to make a fraction of the steps of a gait; for the other part of the joint movement, the actuator remains inactive, and the weight of the prosthesis can be exploited to complete the movement.

Due to this mode of operation of the prosthesis, the actuator is not always active, and it is therefore possible to save battery power by exploiting the cycle phase during which it is not active.

In the preferential solution disclosed in American patent application US 2010/0023133, the actuator is a hydraulic one and operates by means of a hydraulic circuit wherein an electric motor drives a pump that delivers oil to the actuator through a flow control circuit comprising several valves (e.g. two-way or three-way valves, non-return valves, etc.) according to many different feasible construction variants, as described in the above-mentioned document.

Advantageously, the hydraulic flow control system permits controlling a regenerative phase, wherein the fluid circulating in the circuit can drive the pump, which is a reversible one, so that hydraulic energy can be recovered to recharge the electric batteries.

While the prosthesis known from American patent application US 2010/0023133 has, from a general viewpoint, some interesting features, such as those summarized above, it does not, however, appear to be quite as effective from an application viewpoint.

In the first place, it must be pointed out that the hydraulic actuator acting between the two articulated parts of the prosthesis in order to adjust the torque thereof during the knee flexion and extension phases is controlled by a hydraulic power unit and a valve circuit.

These components imply some construction complexity and also a non-linear operation of the actuator, caused by the inevitable transients occurring when the valves controlled by the respective power unit open and close, as well as by the different resistances (i.e. load losses) encountered by the fluid along the tortuous path in the various branches of the hydraulic circuit.

It should be noted that, in the prosthesis described in the above-mentioned prior-art document, solenoid valves are employed which require electric energy to be controlled, which energy is supplied by batteries; this solution does not appear to be optimal with a view of ensuring a longer operating time of the prosthetic device.

Lastly, the fluid delivery pump comprises gears that seem to contribute, together with the valves and the hydraulic circuit, to generating inevitable noise, which should preferably be reduced.

Based on this analysis, it can be stated that a technical problem addressed by the invention is to provide a hydraulic actuator and a limb prosthesis comprising such actuator whose features make it possible to overcome the limitations of the above-described state of the art.

Although the prosthesis is preferably intended for a lower limb, in particular the knee, the invention is also applicable to the upper limbs, while the actuator involved may be useful also for other devices not necessarily intended for use as rehabilitating or aiding devices for the human body.

Within the scope of this general technical problem, it is a more specific object of the invention to provide a high-efficiency, small-size actuator that can advantageously be used in wearable or portable devices for humans.

It is another object of the invention to provide an actuator having low power consumption and noise, thus being suitable for prosthetic devices, which will benefit from a longer operating time, all other conditions being equal, than prior-art devices.

The idea that solves the above-mentioned problem and that permits achieving the above-mentioned objects is to provide a linear electro-hydrostatic actuator (EHA) which is fully integrated and optimized for a motorized limb prosthesis.

The specific features of the EHA actuator developed in accordance with the invention provide the prosthesis with both assistive power (active operation) and passive power, i.e. energy collection (regenerative operation).

In this manner, the final device, i.e. the prosthetic leg, will allow the amputee to execute more complex tasks in a more natural way. Moreover, it will increase the autonomy of the device, so that the user will have the possibility of using it longer during the day.

Patent document US2010/023133 A1 discloses a controlled powered knee prosthesis. Patent document CN110202608 discloses the use of an hydraulic actuator to rotate the joint of a robot.

The features of the invention are more specifically set out in the claims appended to this description.

Such features will become more apparent in the light of one possible example of embodiment of the invention, which will be described hereinafter with reference to the annexed drawings, wherein:

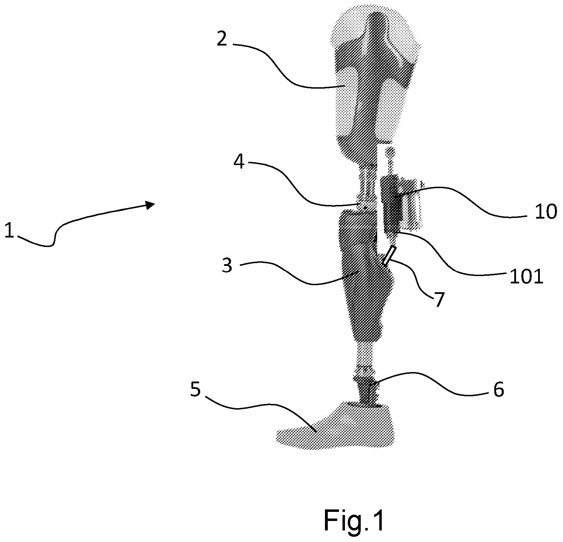

Fig. 1 shows a lower-limb prosthesis in accordance with the invention;

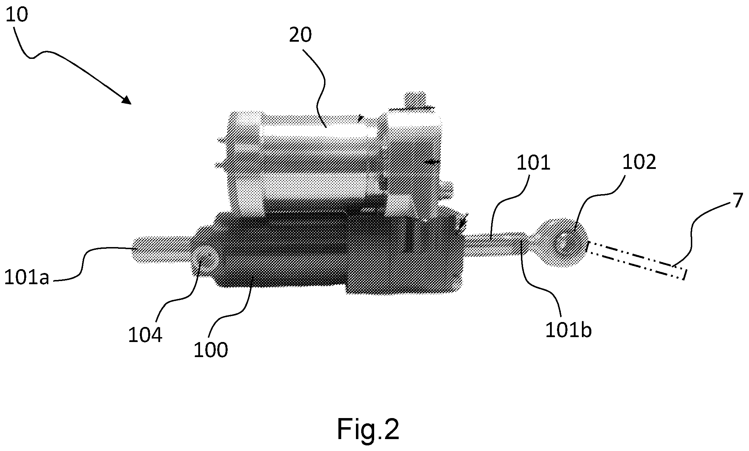

Fig. 2 shows a linear electro-hydrostatic actuator according to the invention;
Fig. 3 shows a detail of the electro-hydrostatic actuator of the assembly of Figure 2;
Fig. 4 is a longitudinal section of the actuator of Figure 3;
Fig. 5 shows a cross-section of a volumetric pump of the electro-hydrostatic actuator of Fig. 2;
Fig. 6 is a diagram that illustrates the operation of the pump of Fig. 5;

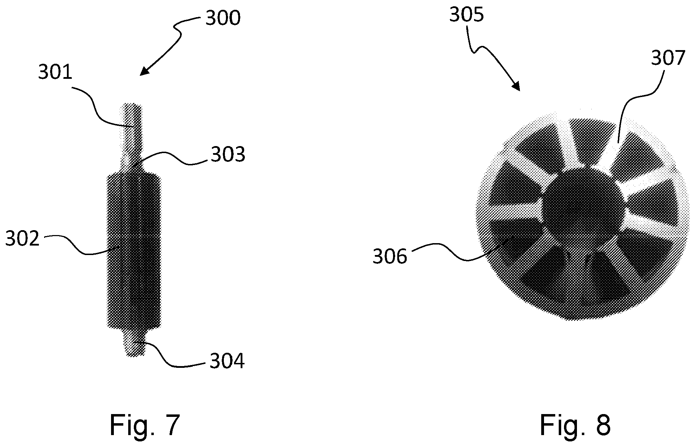

Figs. 7 and 8 show, respectively, a longitudinal view of the shaft and a front view of the stator of the motor that drives the pump of Fig. 5;
Fig. 9 is a diagram that illustrates the electro-hydrostatic actuator in accordance with the invention.

With reference to the above-listed drawings, 1 designates as a whole a lower-limb prosthesis in accordance with the invention.

The prosthesis 1 comprises an upper first part 2, which is applied to the thigh of an amputee, and a lower second part 3 corresponding to the leg or tibial tract of the lower limb, to which a ground supporting base or member 5 is connected which acts as a foot.

The upper part 2 and the lower part 3 of the prosthesis 1 are connected to each other in an articulated manner, so as to be able to make mutual rotations, by means of a knee articulation essentially configured as a hinge; the rotation of the parts 2 and 3 of the prosthesis occurs in a plane, though other more complex embodiments are also possible, e.g. ball-joint solutions allowing for spatial rotations.

In accordance with a preferred embodiment of the prosthesis 1, the supporting end or foot 5 is connected to the lower or tibial part 3 in a manner such as to form an ankle joint; the solutions adopted for making this ankle joint may be similar to those (hinge or ball joint) employed for the knee, or else they may be different and specifically designed for this purpose.

In this respect, it must be pointed out that any reference to "an embodiment" or "an implementation" in this description will indicate that a particular configuration, structure or feature is comprised in at least one embodiment of the invention. Therefore, the expression "in one embodiment" and the like, which can be found in several parts of this description, will not necessarily refer to the same embodiment. Moreover, those skilled in the art will understand that any particular configurations, structures or features may be combined as deemed appropriate to attain the results explained herein, and that the alphanumerical references in the drawings only serve to clarify the description and shall not limit the protection scope or extent of the various embodiments.

In light of this clarification, the prosthesis 1 comprises an electro-hydrostatic actuator 10 acting between the upper part 2 and the lower part 3 to assist the knee articulation 4 during the flexion and extension movements.

In brief, the linear electro-hydrostatic actuator 10 consists, in this case, of a combination of three sub-components: an electric motor, a hydraulic pump and a linear hydraulic cylinder.

The electric motor outputs a torque to rotate the hydraulic pump, which generates a pressure rise and produces a volumetric flow that translates into translational force and velocity of the linear hydraulic actuator.

These actuators are usually employed in the aircraft industry, because they allow such parts as wing flaps and undercarriage to be remotely controlled without requiring a hydraulic system with tubes, valves and a separate pump, but simply an electric connection.

To the Applicants' knowledge, no applications of electro-hydrostatic actuators for prosthetic devices or for movement aiding equipment for humans (e.g. exoskeletons) exist yet.

In order to achieve this object, in the example of the invention shown in the drawings the electro-hydrostatic actuator 10 comprises a hydraulic cylinder 100, with which a rod 101 is associated which develops in the longitudinal direction, protruding from both sides with its ends 101a, 101b.

The first end 101a is free, since it is not connected to any other part of the prosthesis, whereas the second end 101b of the rod is connected to the upper part 2 of the prosthesis via the ball joint 102.

The connection of the electro-hydrostatic actuator to the lower part 3 of the prosthesis is effected by means of bushings 104 provided in the hydraulic cylinder 100.

The ends of the rod 101a, 101b may be configured as deemed appropriate, e.g. with threads, holes, etc., for mounting connection elements, such as the ring or ball joint 102 in the case shown in the drawings.

The ring 102 is intended to engage with an articulated element 7, e.g. a connecting rod, a crank, a hinge, or the like, connected to the corresponding upper part 2 of the prosthesis to transmit either an active (motive) torque or a passive (resistant) torque as a function of the flexion and extension movements of the prosthesis 1.

The hydraulic cylinder 100 comprises an external structure or casing 103, where there is a cylindrical chamber 105 in which a piston 107, mounted to the rod 101, can move. Advantageously, the piston 107 is a double-acting one and moves to and fro in the cylindrical chamber 105 under the action of the oil supplied into the hydraulic cylinder 100 through the inlet and outlet ports 110, 111.

The latter are used for letting the oil enter and exit the hydraulic cylinder 100 according to the different operating phases of the actuator 1, in coordination with a volumetric pump 20, which will be further described hereafter.

To this end, the casing 103 features ducts or channels (not shown in the drawings) through which the oil flows, and which lead into the internal cylindrical chamber 105 on opposite sides of the piston 107.

In accordance with a preferred embodiment, the electro-hydrostatic actuator 10 comprises no oil accumulation chambers or tanks, either inside or outside the casing 103 of the hydraulic cylinder 100: this ensures better quickness and a symmetrical behaviour in the movements of the piston 107.

An important contribution to this effect is given by the volumetric pump 20, which, in accordance with a preferred embodiment of the invention, is of the generated rotor, also known as "gerotor", type.

Although this pump type is known as concerns its general characteristics (see, for example, American patent publications US 3,453,966 and US 4,501,536), no applications of such pumps for prosthetic devices or movement aiding equipment for humans exist yet.

A gerotor is a volumetric pump (the term "gerotor" derives from "generated rotor") essentially consisting of an internal rotor 200 and an external rotor 201 forming an epicyclic gearing.

The internal rotor 200 has a number n of teeth, while the external rotor 201 has n+1 teeth, with n defined as a natural number greater than or equal to 2. The axis of the internal rotor 200 is offset by an eccentricity "E" from the axis of the external rotor 201, and both rotors rotate about their respective axes.

The geometry of the two rotors 200, 201 divides the space between them into a series of different dynamically-changing volumes V₁, V₂, V₃...Vₙ: during the rotation, each one of these volumes changes continuously, first increasing and then decreasing.

Tight tolerances and high precision of the profile of the rotors' teeth permit obtaining a sealed separation between the suction and delivery volumes V of the pump.

Thus, the volume increase creates a vacuum, which in turn results in suction of the fluid; this part of the cycle occurs as the fluid enters the pump.

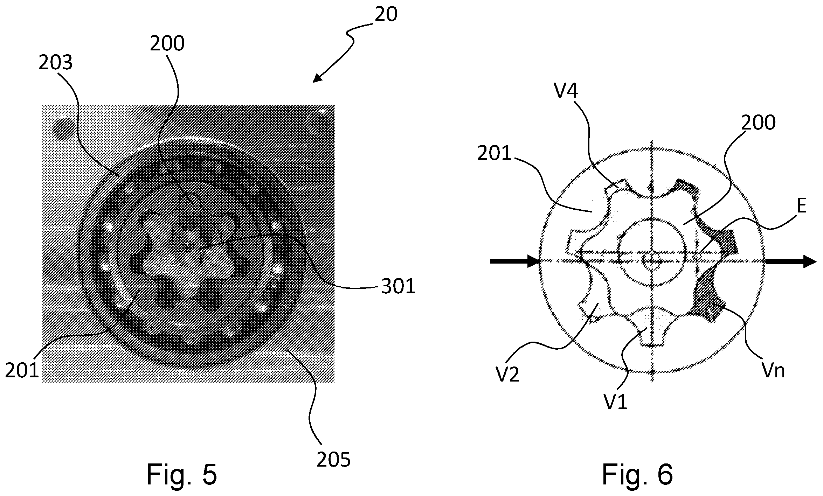

As the volume V decreases, the fluid in that space is compressed, and can then be pumped or, if it is a gaseous fluids, compressed; this situation is shown schematically in Figure 6, wherein the pump suction side is on the left and the pump delivery side is on the right (in black), and the fluid direction is indicated by the arrows.

In this exemplary embodiment, the internal rotor 200 is coupled to a pinion 301 at the end of the shaft 301 of the electric motor 30 that drives the pump 20, while the external rotor 201 is supported within the body 205 of the pump and can roll freely on ball bearings 203.

This solution reduces the friction of the volumetric pump 20, making it particularly suitable for application to the prosthesis 1 because it contributes to reducing the power consumption of the motor 30 that drives the pump 20.

In accordance with a preferred embodiment of the invention, the motor 30 is a permanent-magnet brushless motor that ensures high performance while taking up little space.

With this in mind, in this example the geometry of the motor was analytically derived starting from physical constraints in terms of performance and installation, and was optimized by FEM simulation to maximize the output torque.

This resulted in a stator 305 using windings 306 concentrated in trapezoidal cavities 307 to reduce the overall length to a minimum, thus facilitating integration and reducing Joule losses and production complexity.

Moreover, the shaft 300 also has a compact configuration, wherein the permanent magnets 302 are arranged between the support ends 303 and 304 fitted with bearings (not shown in the drawings), one of which is equipped with a projecting pinion 301, whereon the internal rotor 200 of the pump 20 is fitted.

In order to operate, the prosthesis 1 equipped with the electro-hydrostatic actuator 10 according to the invention requires, in addition to electric batteries to be worn by the user, a number of sensors and at least one control unit (CPU or the like), to which the sensors and the various components such as the hydraulic cylinder 100, the pump 20, the electric motor 30, etc. are connected (preferably via wireless connections, e.g. Wi-Fi). All these items are not shown in the drawings, since those skilled in the art will be able to appropriately arrange them while taking into account the following explanations. During the flexion phase of the knee articulation 4, i.e. the phase wherein the upper part 2 and the lower part 3 of the prosthesis 1 make a relative rotation that brings them closer to each other, the actuator 10 applies an active torque to the element 7, so as to move it as a function of the rotation that the parts 2, 3 of the prosthesis must make.

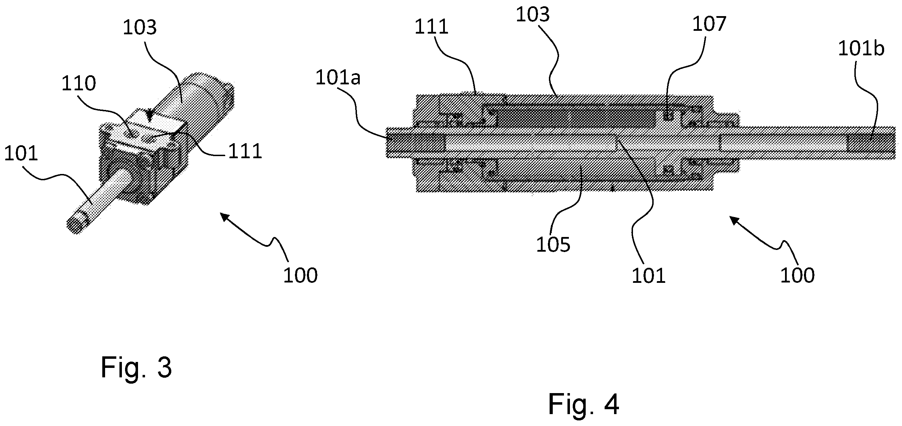

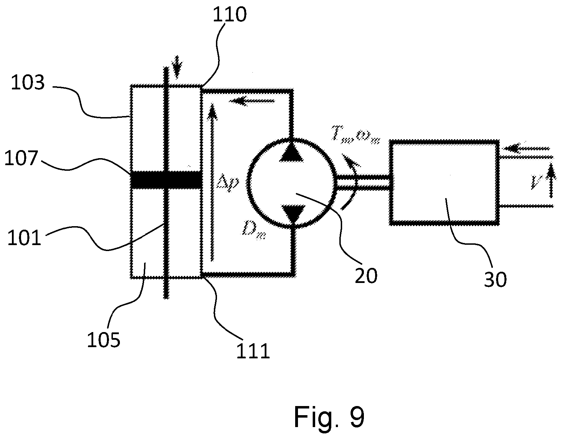

For this purpose, the pump 20 supplies oil into the cylindrical chamber 105 through one of the ports 110, 111, thus causing the piston 107 to move in one direction (to the left or to the right in Figure 4, or upwards or downwards in Figure 9).

Following the movement of the piston 107, the same quantity of fluid flows out of the cylindrical chamber 105 through the other one of the ports 110, 111, since the system is advantageously reversible and has a symmetric configuration, as can be seen in the diagram of Figure 9.

In particular, it must be pointed out that the use of a volumetric pump 20, in particular a rotary pump, preferably like the generated rotor pump (gerotor) of the example illustrated herein, advantageously permits feeding the fluid in a reversible manner in either direction within the hydraulic cylinder 100 (through the ports 110, 111) simply by reversing the direction of rotation of the motor 30.

It is thus no longer necessary to include valves in the hydraulic circuit, as was the case in the prior art, for deviating the fluid into one part or the other of the hydraulic actuator.

As aforesaid, the operation of the actuator 10 according to the invention may envisage both an active phase, wherein the actuator exerts a force on the parts 2, 3 of the prosthesis in order to cause them to move, and a passive or regenerative phase, wherein the parts 2, 3 of the prosthesis induce (e.g. by gravity) a backward movement of the actuator 10, so that the piston 107 inside of it will make a return stroke, thereby causing the oil to flow back from the cylindrical chamber 105 in the reverse direction.

This oil backflow will cause the rotors 200, 201 of the pump 20 to turn, thus supplying mechanical energy to the electric motor 30, which during this phase will operate as a generator in order to recharge the batteries.

All these operating phases of the actuator 10 are managed by a control system, to which all the components taken into account herein (electric motor 30, pump 20, etc.) are connected, in addition to a network of sensors necessary for monitoring all the operating parameters (e.g. power supply voltage V, torque Tₘ and revolution speed ωₘ of the motor 30, pump displacement Dₘ, fluid pressure difference Δp between the inlet and the outlet of the hydraulic cylinder 100, etc.).

The above explanation of the operation of the electro-hydrostatic actuator 10 also applies, *mutatis mutandis,* to the extension phase of the articulation 4 of the prosthesis 1, wherein the upper part 2 and the lower part 3 rotate relative to each other to arrange themselves into a substantially aligned condition.

Therefore, the electro-hydrostatic actuator 10 behaves as a device capable of evenly supplying a controlled force or torque to the prosthesis 1 during both phases of flexion and extension of the prosthetic limb.

Furthermore, the teaching of the invention permits achieving several important advantages.

The first one is a higher global efficiency of the overall kinematic chain, compared with gear-based drives, e.g. harmonic drives or epicyclic gearings.

Secondly, it is reversible; this is another key feature for robotic and prosthetic systems, wherein the devices should be able to safely interact with both the user and the environment.

Thirdly, it is easily controllable, since the whole control part is executed on the electric motor side, thus avoiding the use of inefficient and typically bulky valve-based hydraulic control systems.

The linear drive of the lower-limb prosthesis ensures a better distribution of the actuator's mass along the vertical axis of the leg, thus better approaching a physiological distribution of the limb's mass.

This advantage surpasses all gear-based rotary-drive prosthetic devices, which accumulate most of the device's mass around the knee axis, thus generating discomfort.

Moreover, the use of a linear hydrostatic actuation ensures a variable transmission ratio that can be optimally adjusted to better suit the patient's biomechanical needs; the reversible actuation of the device makes it possible to maximize the collaboration among the prosthesis, the user and the environment.

The implementation of an EHA with no hydraulic valves or accumulators ensures a very rigid transmission, with no additional losses between the motor-pump assembly and the hydraulic actuator. This increases the efficiency of the system and makes the device more controllable.

Further features of the invention include the side-by-side arrangement of the hydraulic cylinder 100 and the body 205 with respect to the pump 20, resulting also in a minimal length of the fluid path between these two components in both directions: this produces a compact configuration of the electro-hydrostatic actuator 1, making it particularly suitable for application to prosthetic devices or movement aiding equipment for humans. All of these features fall within the scope of the following claims.

## Claims

1. Electro-hydrostatic actuator for a joint prosthesis, comprising a hydraulic cylinder (100) including at least one cylindrical chamber (105) and a piston (107) that is movable in such cylindrical chamber (105), a pump (20) in fluidic communication with the hydraulic cylinder (100) for supplying a fluid into the cylindrical chamber (105), **characterized in that** the pump (20) is of the rotary volumetric type.

2. Actuator according to claim 1, wherein the volumetric pump (20) is of the generated rotor type (200, 201).

3. Actuator according to claim 2, wherein the generated rotor pump (20) comprises an internal rotor (200) and an external rotor (201) supported by rolling means.

4. Actuator according to claims 1, 2 or 3, wherein the fluidic communication between the hydraulic cylinder (100) and the pump (20) for supplying a fluid into the cylindrical chamber (105) occurs directly without using any valves or other fluid interception devices.

5. Actuator according to any one of claims 1 to 4, wherein the piston (107) is a double-acting one and the fluidic communication path between the hydraulic cylinder (100) and the pump (20) is substantially symmetrical.

6. Actuator according to any one of claims 1 to 5, wherein the hydraulic cylinder (100) and the pump (20) are arranged side by side.

7. Prosthesis for aiding the human body, comprising first and second parts (2, 3), an articulation (4) mutually connecting said first and second parts (2, 3) in a manner such that they can make rotations relative to each other, an electro-hydrostatic actuator (EHA) (10) adapted to control said rotations, **characterized in that** the electro-hydrostatic actuator (10) is according to any of claims 1 to 6.

8. Prosthesis according to claim 7, wherein the volumetric pump (20) is a rotary one.

9. Prosthesis according to claims 7 or 8, wherein the volumetric pump (20) is of the generated rotor type (200, 201).

10. Prosthesis according to any one of the preceding claims 7 to 9, wherein the electro-hydrostatic actuator (10) is active between said articulated parts (2, 3).

11. Prosthesis according to claim 10, comprising a rod (101) associated with a hydraulic cylinder (100), movable in the longitudinal direction and connected to said articulated parts (2, 3) to actuate the rotations thereof.

12. Prosthesis according to any one of the preceding claims 7 to 11, wherein the electro-hydrostatic actuator (10) comprises a hydraulic cylinder (100) in fluidic communication with a rotary volumetric pump (20), which is adapted to supply the fluid into the hydraulic cylinder (100) in opposite directions by reversing its direction of rotation.

13. Prosthesis according to claim 12, wherein the rotary volumetric pump (20) is associated with an electric motor (30) and is adapted to operate in a regenerative manner for generating electric energy through the electric motor (30).

## Patentansprüche

1. Elektrohydraulischer Aktuator für eine Gelenksprothese, umfassend einen Hydraulikzylinder (100), der zumindest eine zylindrische Kammer (105) und einen Kolben (107), der in der zylindrischen Kammer (105) beweglich ist, beinhaltet, und eine Pumpe (20) in Fluidverbindung mit dem Hydraulikzylinder (100), um ein Fluid in die zylindrische Kammer (105) zuzuführen, **dadurch gekennzeichnet, dass** die Pumpe (20) vom drehvolumetrischen Typ ist.

2. Aktuator nach Anspruch 1, wobei die volumetrische Pumpe (20) vom Gerotor Typ (200, 201) ist.

3. Aktuator nach Anspruch 2, wobei die Gerotorpumpe (20) einen inneren Rotor (200) und einen äußeren Rotor (201) umfasst, die durch Rollmittel gelagert sind.

4. Aktuator nach den Ansprüchen 1, 2 oder 3, wobei die Fluidverbindung zwischen dem Hydraulikzylinder (100) und der Pumpe (20) zum Zuführen eines Fluids in die zylindrische Kammer (105) direkt ohne Verwendung von irgendwelchen Ventilen oder anderen Fluidstoppvorrichtungen erfolgt.

5. Aktuator nach einem der Ansprüche 1 bis 4, wobei der Kolben (107) ein doppeltwirkender ist und der Fluidverbindungspfad zwischen dem Hydraulikzylinder (100) und der Pumpe (20) im Wesentlichen symmetrisch ist.

6. Aktuator nach einem der Ansprüche 1 bis 5, wobei der Hydraulikzylinder (100) und die Pumpe (20) nebeneinander angeordnet sind.

7. Prothese zur Unterstützung des menschlichen Körpers, umfassend einen ersten und einen zweiten Teil (2, 3), ein Gelenk (4), das den ersten und den zweiten Teil (2, 3) auf solche Weise miteinander verbindet, dass diese Drehungen in Bezug aufeinander ausführen können, und ein elektrohydraulischer Aktuator (EHA) (10), das dazu geeignet ist, diese Drehungen zu steuern, **dadurch gekennzeichnet, dass** der elektrohydraulische Aktuator (10) eines nach einem der Ansprüche 1 bis 6 ist.

8. Prothese nach Anspruch 7, wobei die volumetrische Pumpe (20) eine drehende ist.

9. Prothese nach den Ansprüchen 7 oder 8, wobei die volumetrische Pumpe (20) vom Gerotor Typ (200, 201) ist.

10. Prothese nach einem der vorhergehenden Ansprüche 7 bis 9, wobei das elektrohydraulischer Aktuator (10) zwischen den aneinander angelenkten Teilen (2, 3) aktiv ist.

11. Prothese nach Anspruch 10, umfassend eine Stange (101), die einem Hydraulikzylinder (100) zugeordnet, in der Längsrichtung beweglich und mit den angelenkten Teilen (2, 3) verbunden ist, um die Drehbewegungen derselben zu betätigen.

12. Prothese nach einem der vorhergehenden Ansprüche 7 bis 11, wobei das elektrohydraulischer Aktuator (10) einen Hydraulikzylinder (100) in Fluidverbindung mit einer drehvolumetrischen Pumpe (20) umfasst, welche dazu geeignet ist, das Fluid in entgegengesetzten Richtungen in den Hydraulikzylinder (100) zuzuführen, indem sie ihre Drehrichtung umkehrt.

13. Prothese nach Anspruch 12, wobei die drehvolumetrische Pumpe (20) einem Elektromotor (30) zugeordnet ist und dazu geeignet ist, auf regenerative Weise zu arbeiten, um elektrische Energie durch den Elektromotor (30) zu erzeugen.

## Revendications

1. Actionneur électrohydrostatique pour une prothèse articulaire, comprenant un vérin hydraulique (100) comportant au moins une chambre cylindrique (105) et un piston (107) mobile dans ladite chambre cylindrique (105), une pompe (20) en communication fluidique avec le vérin hydraulique (100) pour fournir un fluide dans la chambre cylindrique (105), **caractérisé en ce que** la pompe (20) est du type volumétrique rotatif.

2. Actionneur selon la revendication 1, dans lequel la pompe volumétrique (20) est du type pompe à gérotor (200, 201).

3. Actionneur selon la revendication 2, dans lequel la pompe à gérotor (20) comprend un rotor interne (200) et un rotor externe (201) supportés par des moyens de roulement.

4. Actionneur selon les revendications 1, 2 ou 3, dans lequel la communication fluidique entre le vérin hydraulique (100) et la pompe (20) pour alimenter en fluide la chambre cylindrique (105) s'effectue directement sans utiliser de vannes ou d'autres dispositifs d'interception de fluide.

5. Actionneur selon l'une quelconque des revendications 1 à 4, dans lequel le piston (107) est à double effet et le chemin de communication fluidique entre le vérin hydraulique (100) et la pompe (20) est sensiblement symétrique.

6. Actionneur selon l'une quelconque des revendications 1 à 5, dans lequel le vérin hydraulique (100) et la pompe (20) sont disposés côte à côte.

7. Prothèse destinée à aider le corps humain, comprenant des première et deuxième parties (2, 3), une articulation (4) reliant mutuellement lesdites première et deuxième parties (2, 3) de manière à ce qu'elles puissent effectuer des rotations l'une par rapport à l'autre, un actionneur électrohydrostatique (EHA) (10) adapté pour commander lesdites rotations, **caractérisée en ce que** l'actionneur électrohydrostatique (10) est selon l'une quelconque des revendications 1 à 6.

8. Prothèse selon la revendication 7, dans laquelle la pompe volumétrique (20) est une pompe rotative.

9. Prothèse selon les revendications 7 ou 8, dans laquelle la pompe volumétrique (20) est du type pompe à gérotor (200, 201).

10. Prothèse selon l'une quelconque des revendications 7 à 9 précédentes, dans laquelle l'actionneur électrohydrostatique (10) est actif entre lesdites parties articulées (2, 3).

11. Prothèse selon la revendication 10, comprenant une tige (101) associée à un vérin hydraulique (100), mobile dans la direction longitudinale et reliée auxdites parties articulées (2, 3) pour actionner leurs rotations.

12. Prothèse selon l'une quelconque des revendications 7 à 11 précédentes, dans laquelle l'actionneur électrohydrostatique (10) comprend un vérin hydraulique (100) en communication fluidique avec une pompe volumétrique rotative (20), qui est adaptée pour fournir le fluide dans le vérin hydraulique (100) dans des directions opposées en inversant son sens de rotation.

13. Prothèse selon la revendication 12, dans laquelle la pompe volumétrique rotative (20) est associée à un moteur électrique (30) et est adaptée pour fonctionner de manière régénérative afin de générer de l'énergie électrique par l'intermédiaire du moteur électrique (30).
